# EUROPEAN PATENT SPECIFICATION

(11) **EP 0 862 622 B1**
(45) Date of publication and mention of the grant of the patent: **18.08.2004**
(21) Application number: 96938081.5
(22) Date of filing: 05.11.1996
(51) Int. Cl.: C12N 9/12, A61K 38/45, C12Q 1/48, C07K 14/47, A61K 38/17

(54) **Method of screening for RAC protein kinase interacting compounds**
Methode zur Identifizierung von Verbindungen die mit RAC Proteine Kinase interagieren
Méthode de criblage de composés interagissant avec RAC proteine kinase

(30) Priority: 16.11.1995 GB 9523379; 15.12.1995 GB 9525704
(43) Date of publication of application: 09.09.1998
(73) Proprietor: Novartis AG, 4056 Basel (CH); Novartis Pharma GmbH, 1230 Wien (AT)
(72) Inventor: HEMMINGS, Brian, Arthur, CH-4126 Bettingen (CH); COHEN, Philip, Invergowrie DD2 5DQ (GB); ALESSI, Dario, Dundee DD2 2LG (GB); CROSS,Darren, Cheshire SK10 3SH (GB); ANDJELKOVIC, Mirjana, 4054 Basel (CH)
(86) International application number: PCT/EP1996/004810
(87) International publication number: WO 1997/018303

(56) References cited:
- BIOCHEMICAL AND BIOPHYSICAL RESEARCH COMMUNICATIONS, vol. 205, no. 1, 30 November 1994, ORLANDO, FL US, pages 817-825, XP002025952 KONISHI H. ET AL.: "Molecular cloning of rat RAC protein kinase alpha and beta and their association with protein kinase C dseta"
- PROC NATL ACAD SCI U S A 88 (10). 1991. 4171-4175, XP002025953 JONES P F ET AL: "MOLECULAR CLONING AND IDENTIFICATION OF A SERINE THREONINE PROTEIN KINASE OF THE SECOND-MESSENGER SUBFAMILY." cited in the application
- NATURE (LONDON) 378 (6559). 1995. 785-789., XP002025954 CROSS D A E ET AL: "Inhibition of glycogen synthase kinase -3 by insulin mediated by protein kinase B."
- PROCEEDINGS OF THE NATIONAL ACADEMY OF SCIENCES OF USA, vol. 93, no. 12, 11 June 1996, WASHINGTON US, pages 5699-5704, XP002025955 ANDJELKOVIC M. ET AL.: "Activation and phosphorylation of a pleckstrin homology domain containing protein kinase (RAC-PK/PKB) promoted by serum and protein phosphatase inhibitors"

### SEQUENCE LISTING

### (1) GENERAL INFORMATION:

(i) APPLICANT:
   (A) NAME: CIBA-GEIGY AG
   (B) STREET: Klybeckstr. 141
   (C) CITY: Basel
   (E) COUNTRY: Switzerland
   (F) POSTAL CODE (ZIP): 4002
   (G) TELEPHONE: +41 61 69 11 11
   (H) TELEFAX: + 41 61 696 79 76
   (I) TELEX: 962 991
(ii) TITLE OF INVENTION: Compound and method
(iii) NUMBER OF SEQUENCES: 4
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.30 (EPO)

### (2) INFORMATION FOR SEQ ID NO: 1:

(i) SEQUENCE CHARACTERISTICS:
   (A) LENGTH: 1302 base pairs
   (B) TYPE: nucleic acid
   (C) STRANDEDNESS: single
   (D) TOPOLOGY: linear
(ii) MOLECULE TYPE: cDNA to mRNA
(iii) HYPOTHETICAL: NO
(iv) ANTI-SENSE: NO
(v) FRAGMENT TYPE: C-terminal
(ix) FEATURE:
   (A) NAME/KEY: CDS
   (B) LOCATION:2..145
(ix) FEATURE:
   (A) NAME/KEY: repeat_region
   (B) LOCATION:981..1279
(xi) SEQUENCE DESCRIPTION: SEQ ID NO: 1:
   G AAT TCG GCA CGA GCT AGA GCA AGC GCG GCC CCG CGG CCC GGA GCC          46
   ATG CTG AGG AGC TGC GCC GCG CGC CTC CGC ACG CTG GGG GCT CTG TGC        94
   CGG CCG CCA GTA GGC CGG CGC CTG CCG GAA GCG ACC CGC GAC CCG AGC       142
   TGA GGTCATTTTC TTCTGAGGAA GTCATTCTTA AGGACTGTTC TGTCCCCAAC            195
   CCCAGCTGGA ACAAGGACCT AAGACTGCTC TTTGACCAGT TTATGAAGAA ATGTGAAGAT      255
   GGCTCCTGGA AACGTTTGCC TTCATATAAA CGTACACCTA CTGAATGGAT TCAAGACTTC      315
   AAAACCCATT TTCTTGACCC AAAGCTTATG AAAGAAGAAC AAATGTCACA GGCCCAGCTC      375
   TTCACCAGAA GCTTTGATGA TGGCCTGGGC TTTGAATACG TGATGTTCTA CAATGACATT      435
   GAGAAAAGGA TGGTTTGCTT ATTTCAAGGA GGCCCTTACC TGGAAGACCA CCTGGATTCA      495
   TTCATGGAGG TGCCATTGCA ACCATGATTG ATGCTACTGT TGGTATGTGT GCAATGATGG      555
   CTGGGGGAAT CGTCATGACT GCCAATCTCA ACATCAATTA TCCCCGACCT ATCCCTCTTT      615
   GTTCTGTTGT TATGATAAAT AGCCAACTTG ATAAAGTTGA AGGAAGGAAA TTTTTTGTTT      675
   CCTGTAATGT TCAGAGTGTT GATGAGAAGA CCCTATACTC AGAGGCGACA AGCTTATTAT      735
   AAAGTCGAAT CCTGCTAAAA GTCTTGATCG ATAAAGAGTC GTCGGTGAAC TCCATCTCAT      795
   TCTCGCCCCT CCAGAAGAAG GCAGTTGTCC CCCAAATACT CTGCTCCCTC ACTGCTGAAT      855
   CCTGTAGGGA GAAGCCTGCC AACAGTGACC TTCCGAAACA GCCTTCTGAA TACAAAGAGG      915
   ATTCAGTTTC CATCTTCTCA ACTTTTTAAC ACAGAAACAC TTCCTGCGAG ACTATCGACA      975
   ACTCTCGGGC CAGGCGCTGT GGCTCACACC TGTAATCCCA GCACTTTAGG AGGCCGAGGC     1035
   AGGCGGATTG CCTGAGCTCA GGAGTTGAAG ATCAGTCTGG GCAACACGAT GAAACTCCGT     1095
   CTCTACTAAA ATACAAAAAA TTATCCAGGC ATGGTGGCGT ACGCCTGTAG TCCCAGCTAC     1155
   TCAGGAGGCT GAGGCAGGAG AATCGCTTGA ACCCAGGAGG AAGAGGTTGC AGTGAGCCAA     1215
   GATCATGCCA CATCACTCCA ACCTGGGCAA CAGAACAAGA ACCCATCTCA AACAAACAAC     1275
   AAACAAAAAA AAAAAAAAAA ACTCGAG                                         1302

### (2) INFORMATION FOR SEQ ID NO: 2:

(i) SEQUENCE CHARACTERISTICS:
   (A) LENGTH: 48 amino acids
   (B) TYPE: amino acid
   (D) TOPOLOGY: linear
(ii) MOLECULE TYPE: protein
(xi) SEQUENCE DESCRIPTION: SEQ ID NO: 2:

### (2) INFORMATION FOR SEQ ID NO: 3:

(i) SEQUENCE CHARACTERISTICS:
   (A) LENGTH: 2610 base pairs
   (B) TYPE: nucleic acid
   (C) STRANDEDNESS: single
   (D) TOPOLOGY: linear
(ii) MOLECULE TYPE: cDNA to mRNA
(iii) HYPOTHETICAL: NO
(iv) ANTI-SENSE: NO
(vi) ORIGINAL SOURCE:
   (A) ORGANISM: Homo sapiens
(vii) IMMEDIATE SOURCE:
   (B) CLONE: Human RAC alpha
(ix) FEATURE:
   (A) NAME/KEY: CDS
   (B) LOCATION:199..1641
(ix) FEATURE:
   (A) NAME/KEY: mat_peptide
   (B) LOCATION:199..1641
(xi) SEQUENCE DESCRIPTION: SEQ ID NO: 3:
   ATCCTGGGAC AGGGCACAGG GCCATCTGTC ACCAGGGGCT TAGGGAAGGC CGAGCCAGCC       60
   TGGGTCAAAG AAGTCAAAGG GGCTGCCTGG AGGAGGCAGC CTGTCAGCTG GTGCATCAGA      120
   GGCTGTGGCC AGGCCAGCTG GGCTCGGGGA GCGCCAGCCT GAGAGGAGCG CGTGAGCGTC      180
   GCGGGAGCCT CGGGCACC ATG AGC GAC GTG GCT ATT GTG AAG GAG GGT TGG        231
                       Met Ser Asp Val Ala Ile Val Lys Glu Gly Trp
                         1               5                  10
   CTG CAC AAA CGA GGG GAG TAC ATC AAG ACC TGG CGG CCA CGC TAC TTC        279
   Leu His Lys Arg Gly Glu Tyr Ile Lys Thr Trp Arg Pro Arg Tyr Phe
   15 20 25
   CTC CTC AAG AAT GAT GGC ACC TTC ATT GGC TAC AAG GAG CGG CCG CAG 327
   Leu Leu Lys Asn Asp Gly Thr Phe Ile Gly Tyr Lys Glu Arg Pro Gln
   30 35 40
   GAT GTG GAC CAA CGT GAG GCT CCC CTC AAC AAC TTC TCT GTG GCG CAG 375
   Asp Val Asp Gln Arg Glu Ala Pro Leu Asn Asn Phe Ser Val Ala Gln
   45 50 55
   TGC CAG CTG ATG AAG ACG GAG CGG CCC CGG CCC AAC ACC TTC ATC ATC 423
   Cys Gln Leu Met Lys Thr Glu Arg Pro Arg Pro Asn Thr Phe Ile Ile
   60 65 70 75
   CGC TGC CTG CAG TGG ACC ACT GTC ATC GAA CGC ACC TTC CAT GTG GAG 471
   Arg Cys Leu Gln Trp Thr Thr Val Ile Glu Arg Thr Phe His Val Glu
   80 85 90
   ACT CCT GAG GAG CGG GAG GAG TGG ACA ACC GCC ATC CAG ACT GTG GCT 519
   Thr Pro Glu Glu Arg Glu Glu Trp Thr Thr Ala Ile Gln Thr Val Ala
   95 100 105
   GAC GGC CTC AAG AAG CAG GAG GAG GAG GAG ATG GAC TTC CGG TCG GGC 567
   Asp Gly Leu Lys Lys Gln Glu Glu Glu Glu Met Asp Phe Arg Ser Gly
   110 115 120
   TCA CCC AGT GAC AAC TCA GGG GCT GAA GAG ATG GAG GTG TCC CTG GCC 615
   Ser Pro Ser Asp Asn Ser Gly Ala Glu Glu Met Glu Val Ser Leu Ala
   125 130 135
   AAG CCC AAG CAC CGC GTG ACC ATG AAC GAG TTT GAG TAC CTG AAG CTG 663
   Lys Pro Lys His Arg Val Thr Met Asn Glu Phe Glu Tyr Leu Lys Leu
   140 145 150 155
   CTG GGC AAG GGC ACT TTC GGC AAG GTG ATC CTG GTG AAG GAG AAG GCC 711
   Leu Gly Lys Gly Thr Phe Gly Lys Val Ile Leu Val Lys Glu Lys Ala
   160 165 170
   ACA GGC CGC TAC TAC GCC ATG AAG ATC CTC AAG AAG GAA GTC ATC GTG 759
   Thr Gly Arg Tyr Tyr Ala Met Lys Ile Leu Lys Lys Glu Val Ile Val
   175 180 185
   GCC AAG GAC GAG GTG GCC CAC ACA CTC ACC GAG AAC CGC GTC CTG CAG 807
   Ala Lys Asp Glu Val Ala His Thr Leu Thr Glu Asn Arg Val Leu Gln
           190                 195                 200
   AAC TCC AGG CAC CCC TTC CTC ACA GCC CTG AAG TAC TCT TTC CAG ACC        855
   Asn Ser Arg His Pro Phe Leu Thr Ala Leu Lys Tyr Ser Phe Gln Thr
       205                 210                 215
   CAC GAC CGC CTC TGC TTT GTC ATG GAG TAC GCC AAC GGG GGC GAG CTG        903
   His Asp Arg Leu Cys Phe Val Met Glu Tyr Ala Asn Gly Gly Glu Leu
   220                 225                 230                 235
   TTC TTC CAC CTG TCC CGG GAA CGT GTG TTC TCC GAG GAC CGG GCC CGC        951
   Phe Phe His Leu Ser Arg Glu Arg Val Phe Ser Glu Asp Arg Ala Arg
                   240                 245                 250
   TTC TAT GGC GCT GAG ATT GTG TCA GCC CTG GAC TAC CTG CAC TCG GAG        999
   Phe Tyr Gly Ala Glu Ile Val Ser Ala Leu Asp Tyr Leu His Ser Glu
               255                 260                 265
   AAG AAC GTG GTG TAC CGG GAC CTC AAG CTG GAG AAC CTC ATG CTG GAC       1047
   Lys Asn Val Val Tyr Arg Asp Leu Lys Leu Glu Asn Leu Met Leu Asp
           270                 275                 280
   AAG GAC GGG CAC ATT AAG ATC ACA GAC TTC GGG CTG TGC AAG GAG GGG       1095
   Lys Asp Gly His Ile Lys Ile Thr Asp Phe Gly Leu Cys Lys Glu Gly
       285                 290                 295
   ATC AAG GAC GGT GCC ACC ATG AAG ACC TTT TGC GGC ACA CCT GAG TAC       1143
   Ile Lys Asp Gly Ala Thr Met Lys Thr Phe Cys Gly Thr Pro Glu Tyr
   300                 305                 310                 315
   CTG GCC CCC GAG GTG CTG GAG GAC AAT GAC TAC GGC CGT GCA GTG GAC       1191
   Leu Ala Pro Glu Val Leu Glu Asp Asn Asp Tyr Gly Arg Ala Val Asp
                   320                 325                 330
   TGG TGG GGG CTG GGC GTG GTC ATG TAC GAG ATG ATG TGC GGT CGC CTG       1239
   Trp Trp Gly Leu Gly Val Val Met Tyr Glu Met Met Cys Gly Arg Leu
               335                 340                 345
   CCC TTC TAC AAC CAG GAC CAT GAG AAG CTT TTT GAG CTC ATC CTC ATG       1287
   Pro Phe Tyr Asn Gln Asp His Glu Lys Leu Phe Glu Leu Ile Leu Met
           350                 355                 360
   GAG GAG ATC CGC TTC CCG CGC ACG CTT GGT CCC GAG GCC AAG TCC TTG      1335
   Glu Glu Ile Arg Phe Pro Arg Thr Leu Gly Pro Glu Ala Lys Ser Leu
       365                 370                 375
   CTT TCA GGG CTG CTC AAG AAG GAC CCC AAG CAG AGG CTT GGC GGG GGC      1383
   Leu Ser Gly Leu Leu Lys Lys Asp Pro Lys Gln Arg Leu Gly Gly Gly
   380                 385                 390                 395
   TCC GAG GAC GCC AAG GAG ATC ATG CAG CAT CGC TTC TTT GCC GGT ATC      1431
   Ser Glu Asp Ala Lys Glu Ile Met Gln His Arg Phe Phe Ala Gly Ile
                   400                 405                 410
   GTG TGG CAG CAC GTG TAC GAG AAG AAG CTC AGC CCA CCC TTC AAG CCC      1479
   Val Trp Gln His Val Tyr Glu Lys Lys Leu Ser Pro Pro Phe Lys Pro
               415                 420                 425
   CAG GTC ACG TCG GAG ACT GAC ACC AGG TAT TTT GAT GAG GAG TTC ACG      1527
   Gln Val Thr Ser Glu Thr Asp Thr Arg Tyr Phe Asp Glu Glu Phe Thr
           430                 435                 440
   GCC CAG ATG ATC ACC ATC ACA CCA CCT GAC CAA GAT GAC AGC ATG GAG      1575
   Ala Gln Met Ile Thr Ile Thr Pro Pro Asp Gln Asp Asp Ser Met Glu
       445                 450                 455
   TGT GTG GAC AGC GAG CGC AGG CCC CAC TTC CCC CAG TTC TCC TAC TCG      1623
   Cys Val Asp Ser Glu Arg Arg Pro His Phe Pro Gln Phe Ser Tyr Ser
   460                 465                 470                 475
   GCC AGC AGC ACG GCC TGA GGCGGCGGTG GACTGCGCTG GACGATAGCT             1671
   Ala Ser Ser Thr Ala  *
                   480
   TGGAGGGATG GAGAGGCGGC CTCGTGCCAT GATCTGTATT TAATGGTTTT TATTTCTCGG    1731
   GTGCATTTGA GAGAAGCCAC GCTGTCCTCT CGAGCCCAGA TGGAAAGACG TTTTTGTGCT    1791
   GTGGGCAGCA CCCTCCCCCG CAGCGGGGTA GGGAAGAAAA CTATCCTGCG GGTTTTAATT    1851
   TATTTCATCC AGTTTGTTCT CCGGGTGTGG CCTCAGCCCT CAGAACAATC CGATTCACGT    1911
   AGGGAAATGT TAAGGACTTC TACAGCTATG CGCAATGTGG CATTGGGGGG CCGGGCAGGT    1971
   CCTGCCCATG TGTCCCCTCA CTCTGTCAGC CAGCCGCCCT GGGCTGTCTG TCACCAGCTA    2031
   TCTGTCATCT CTCTGGGGCC CTGGGCCTCA GTTCAACCTG GTGGCACCAG ATGCAACCTC 2091
   ACTATGGTAT GCTGGCCAGC ACCCTCTCCT GGGGGTGGCA GGCACACAGC AGCCCCCCAG 2151
   CACTAAGGCC GTGTCTCTGA GGACGTCATC GGAGGCTGGG CCCCTGGGAT GGGACCAGGG 2211
   ATGGGGGATG GGCCAGGGTT TACCCAGTGG GACAGAGGAG CAAGGTTTAA ATTTGTTATT 2271
   GTGTATTATG TTGTTCAAAT GCATTTTGGG GGTTTTTAAT CTTTGTGACA GGAAAGCCCT 2331
   CCCCCTTCCC CTTCTGTGTC ACAGTTCTTG GTGACTGTCC CACCGGAGCC TCCCCCTCAG 2391
   ATGATCTCTC CACGGTAGCA CTTGACCTTT TCGACGCTTA ACCTTTCCGC TGTCGCCCCA 2451
   GGCCCTCCCT GACTCCCTGT GGGGGTGGCC ATCCCTGGGC CCCTCCACGC CTCCTGGCCA 2511
   GACGCTGCCG CTGCCGCTGC ACCACGGCGT TTTTTTACAA CATTCAACTT TAGTATTTTT 2571
   ACTATTATAA TATAATATGG AACCTTCCCT CCAAATTCT 2610

### (2) INFORMATION FOR SEQ ID NO: 4:

(i) SEQUENCE CHARACTERISTICS:
   (A) LENGTH: 481 amino acids
   (B) TYPE: amino acid
   (D) TOPOLOGY: linear
(ii) MOLECULE TYPE: protein
(xi) SEQUENCE DESCRIPTION: SEQ ID NO: 4:
   Met Ser Asp Val Ala Ile Val Lys Glu Gly Trp Leu His Lys Arg Gly
    1               5                  10                  15
   Glu Tyr Ile Lys Thr Trp Arg Pro Arg Tyr Phe Leu Leu Lys Asn Asp
               20                  25                  30
   Gly Thr Phe Ile Gly Tyr Lys Glu Arg Pro Gln Asp Val Asp Gln Arg
           35                  40                  45
   Glu Ala Pro Leu Asn Asn Phe Ser Val Ala Gln Cys Gln Leu Met Lys
       50                  55                  60
   Thr Glu Arg Pro Arg Pro Asn Thr Phe Ile Ile Arg Cys Leu Gln Trp
   65 70 75 80
   Thr Thr Val Ile Glu Arg Thr Phe His Val Glu Thr Pro Glu Glu Arg
   85 90 95
   Glu Glu Trp Thr Thr Ala Ile Gln Thr Val Ala Asp Gly Leu Lys Lys
   100 105 110
   Gln Glu Glu Glu Glu Met Asp Phe Arg Ser Gly Ser Pro Ser Asp Asn
   115 120 125
   Ser Gly Ala Glu Glu Met Glu Val Ser Leu Ala Lys Pro Lys His Arg
   130 135 140
   Val Thr Met Asn Glu Phe Glu Tyr Leu Lys Leu Leu Gly Lys Gly Thr
   145 150 155 160
   Phe Gly Lys Val Ile Leu Val Lys Glu Lys Ala Thr Gly Arg Tyr Tyr
   165 170 175
   Ala Met Lys Ile Leu Lys Lys Glu Val Ile Val Ala Lys Asp Glu Val
   180 185 190
   Ala His Thr Leu Thr Glu Asn Arg Val Leu Gln Asn Ser Arg His Pro
   195 200 205
   Phe Leu Thr Ala Leu Lys Tyr Ser Phe Gln Thr His Asp Arg Leu Cys
   210 215 220
   Phe Val Met Glu Tyr Ala Asn Gly Gly Glu Leu Phe Phe His Leu Ser
   225 230 235 240
   Arg Glu Arg Val Phe Ser Glu Asp Arg Ala Arg Phe Tyr Gly Ala Glu
   245 250 255
   Ile Val Ser Ala Leu Asp Tyr Leu His Ser Glu Lys Asn Val Val Tyr
   260 265 270
   Arg Asp Leu Lys Leu Glu Asn Leu Met Leu Asp Lys Asp Gly His Ile
   275 280 285
   Lys Ile Thr Asp Phe Gly Leu Cys Lys Glu Gly Ile Lys Asp Gly Ala
   290 295 300
   Thr Met Lys Thr Phe Cys Gly Thr Pro Glu Tyr Leu Ala Pro Glu Val
   305 310 315 320
   Leu Glu Asp Asn Asp Tyr Gly Arg Ala Val Asp Trp Trp Gly Leu Gly
   325 330 335
   Val Val Met Tyr Glu Met Met Cys Gly Arg Leu Pro Phe Tyr Asn Gln
   340 345 350
   Asp His Glu Lys Leu Phe Glu Leu Ile Leu Met Glu Glu Ile Arg Phe
   355 360 365
   Pro Arg Thr Leu Gly Pro Glu Ala Lys Ser Leu Leu Ser Gly Leu Leu
   370 375 380
   Lys Lys Asp Pro Lys Gln Arg Leu Gly Gly Gly Ser Glu Asp Ala Lys
   385 390 395 400
   Glu Ile Met Gln His Arg Phe Phe Ala Gly Ile Val Trp Gln His Val
   405 410 415
   Tyr Glu Lys Lys Leu Ser Pro Pro Phe Lys Pro Gln Val Thr Ser Glu
   420 425 430
   Thr Asp Thr Arg Tyr Phe Asp Glu Glu Phe Thr Ala Gln Met Ile Thr
   435 440 445
   Ile Thr Pro Pro Asp Gln Asp Asp Ser Met Glu Cys Val Asp Ser Glu
   450 455 460
   Arg Arg Pro His Phe Pro Gln Phe Ser Tyr Ser Ala Ser Ser Thr Ala
   465 470 475 480
   *

## Description

The present invention concerns a method of screening for compounds interacting with RAC protein kinase (RAC-PK).

Protein phosphorylation and dephosphorylation are fundamental processes for the regulation of cellular functions. Protein phosphorylation is prominently involved in signal transduction, where extracellular signals are propagated and amplified by a cascade of protein phosphorylation and dephosphorylation. Two of the best characterised signal transduction pathways involve the c-AMP-dependant protein kinase (PKA) and protein kinase C (PKC). Each pathway uses a different second messenger molecule to activate the protein kinase, which, in turn, phosphorylates specific target molecules.

A novel subfamily of serine/threonine kinases has been recently identified and cloned, termed herein the RAC kinases (RAC-PK: Jones, et al. (1991) Proc. Natl Acad. Sci. USA 88, 4171-4175; Jones, et al. (1991) Cell Regulation 2,1001-1009), but also known as PKB or Akt. RAC kinases have been identified in two closely related isoforms, RACα and RACβ, which share 90% homology at the gene sequence. Mouse RACα (c-akt) is the cellular homologue of the viral oncogene v-akt, generated by fusion of the Gag protein from the AKT8 retrovirus to the N-terminus of murine c-akt. Human RACβ is found to be involved in approximately 10% of ovarian carcinomas, suggesting an involvement of RAC kinases in cell growth regulation.

RAC kinases contain an amino-terminal pleckstrin homology (PH) domain [Hastam, *et al.* (1993) *Nature* **363**, 309-310]. The PH domain was originally identified as an internal repeat, present at the amino and carboxy-termini of pleckstrin, a 47 kDa protein which is the major PKC substrate in activated platelets [Tyers, et al. (1988) *Nature* **333**, 470-473]. The superfamily of PH domain containing molecules consists of over 90 members, including serine/threonine protein kinases (RAC, Nrk, βARK. PKCµ), tyrosine protein kinases (Btk, Tec, Itk), GTPase regulators (ras-GAP, ras-GRF, Vav, SOS, BCR), all known mammalian phospholipase Cs, cytoskeletal proteins (β-spectrin, AFAP-110, syntrophin), "adapter" proteins (GRB-7, 3BP2) and kinase substrates (pleckstrin, IRS-1).

While the PH domain structure has been solved for β-spectrin, dynamin and pleckstrin's amino-terminal domain, its precise function remains unclear. The presence of PH domains in many signalling and cytoskeletal proteins implicates it in mediating protein-protein and membrane interactions. Indeed, the PH domain of the β-adrenergic receptor kinase (βARK) appears partly responsible for its binding to the βγ-subunits of the heterotrimeric G-proteins associated with the β-adrenergic receptor, while the PH domain of the Bruton's tyrosine kinase (Btk) appears to mediate an interaction with PKC. Several PH domains have been shown to be able to bind phosphatidyl-inositol-4-5-bisphosphate *in vitro,* although weakly.

IMPDH is a highly conserved enzyme (41% amino acid identity between bacterial and mammalian sequences) involved in the rate-limiting step of guanine biosynthesis. In mammals there are two isoforms, 84% identical, called type I and type II which are differentially expressed [Natsumeda, *et al.* (1990) *J. Biol. Chem.* **265**, 5292-5295]. Type I is constitutively expressed at low levels while the type II mRNA and protein levels increase during cellular proliferation. IMPDH activity levels are also elevated during rapid proliferation in many cells [Collart, F. R., and Huberman, E. (1988) *J. Biol. Chem.* **263***,* 15769-15772].

By measuring the metabolic fluxes, the proliferative index of intact cancer cells has been shown to be linked with the preferential channelling of IMP into guanylate biosynthesis. Inhibition of cellular IMPDH activity results in an abrupt cessation of DNA synthesis and a cell-cycle block at the G₁-S interface. The specific inhibition of IMPDH by tiazofurin and the subsequent decline in the GTP pool, results in the down regulation of the G-protein ras, which is involved in many signal transduction pathways leading to cellular proliferation (for review see Avruch, *et al.* (1994) *Trends Biochem. Sci.* **19**, 279-283).

Interestingly, p53 has been implicated in regulating IMPDH activity levels [Sherley, J. L. (1991) *J. Biol. Chem.* **266**, 24815-24828]. Here a moderate over-expression of p53 (3-6 fold) induces a profound growth arrest which is rescued by purine nucleotide precursors. Indeed, the p53 over-expression induces a specific block in IMP to XMP conversion, and a diminished activity level of IMPDH. The p53 block does not affect the rate of RNA synthesis, nor is the phenotype rescued by deoxynucleotides indicating that a lack of precursors for DNA synthesis is also not the cause of the block. It would seem most likely that this effect is mediated through a down regulation of the GTP pool required by G-proteins such as ras.

The above observations suggest that IMPDH type II is primarily involved in producing XMP which is channelled into the GTP pool which is crucial for the regulation of G-proteins involved in signal transduction such as ras. It may be that the type I enzyme provides a basal level of XMP that is channelled into the GTP/dGTP pools required for RNA and DNA synthesis. Changes in IMPDH type II activity would alter the GTP/GDP ratio by specifically altering the GTP component which could greatly affect ras signalling pathways as ras is sensitive to small changes in the GTP/GDP ratio.

Glycogen synthase kinase-3 (GSK3) is implicated in the control of several processes important for mammalian cell physiology, including glycogen metabolism and the control of protein synthesis by insulin, as well as the modulation of activity of several transcription factors, such as AP-1 and CREB. GSK3 is inhibited *in vitro* by serine phosphorylation caused by MAP kinase and p70^{S6K}, kinases which lie on distinct insulin-stimulated signalling pathways.

GSK3 is responsible for serine phosphorylation in glycogen synthase, whose dephosphorylation underlies the stimulation of glycogen synthesis by muscle. Thus, GSK3 inactivates glycogen synthase, resulting in an increase in blood sugar levels. Insulin inhibits the action of GSK3, which, in combination with the concomitant activation of phosphatases which dephosphorylate glycogen synthase, leads to the activation of glycogen synthase and the lowering of blood sugar levels.

We have now determined that RAC-PKis directly involved in the inactivation of GSK3 and the activation of IMPDH.

We have used the yeast two-hybrid system [Fields, S. and Song, O-K. (1989) *Nature* **340***,* 245-246; Chien, *et al.* (1991) *Proc. Natl. Acad. Sci. U. S. A*. **88**, 9578-9582] to determine if RAC-PK could function by forming specific interactions with other proteins. We have identified RAC-PK as interacting with human inosine-5' monophosphate dehydrogenase (IMPDH) type II, and with a novel protein termed RAC-PK Carboxy-Terminal Binding Protein (CTBP). RAC-PK stimulates IMPDH Type II activity. In conjunction with the known role of IMPDH in GTP biosynthesis our findings suggest a role for RAC-PK in the regulation of cell proliferation.

Moreover, using a peptide derived from GSK3 and GSK3 itself, we have been able to show that RAC-PK interacts with, phosphorylates and inactivates GSK3. This implicates RAC-PK in the regulation of insulin-dependent signalling pathways, which control cellular proliferation. Taken together, those results suggest a major involvement for RAC-PK in the control of insulin action.

### Summary of the invention

According to the invention, we provide a method of screening for compounds interacting with RAC protein kinase (RAC-PK) comprising the steps of (a) incubating RAC-PK or a functional fragment thereof and the compound to be screened, (b) measuring a kinase activity of RAC-PK or a functional fragment thereof using GSK3 as substrate, and (c) selecting the compound, if said substrate has a different amount of phosphorylated residues as compared to control reaction without said substrate.

### Detailed Description of the Invention.

The invention provides a method of screening for compounds interacting with RAC-PK. The method comprises the steps of (a) incubating RAC-PK or a functional fragment thereof and the compound to be screened, (b) measuring a kinase activity of RAC-PK or a functional fragment thereof using GSK3 as substrate, and (c) selecting the compound, if said substrate has a different amount of phosphorylated residues as compared to control reaction without said substrate.

Vanadate, which term as used herein includes various forms thereof such as ortho- and metavanadate, pervanadate and other rotated vanadium ions, is known as a therapeutic agent in the treatment of diabetes. See, for example, United States Patent 5,421,125. European Patent Application 0 521 787, European Patent Application 0 264 278 and European Patent Application 0 245 979. In UK Patent Application 9525702.8 (Ciba-Geigy AG), filed 15th December 1995, it is disclosed that vanadate is a potent activator of kinases of the insulin-stimulated signalling pathways and of RAC-PK in particular. Accordingly vanadate exerts its therapeutic effect on diabetes by stimulating RAC, which phosphorylates GSK3 and deactivates it, leading to a lowering in blood sugar levels.

RAC-PK and activators thereof are therefore useful in the treatment of diabetes and other diseases where blood sugar levels are excessive. Conversely. inhibitors of RAC-PK such as wortmannin and okadaic acid are useful in the treatment of diseases involving insufficiency in blood sugar levels. Accordingly the present invention provides a method for the screening of compounds useful in the treatment of diseases where blood sugar levels are excessive or of diseases involving insufficiency in blood sugar levels. Preferably, the invention provides a method for the screening of compounds useful in the treatment of diabetes.

In the present invention, RAC protein kinase may be any isoform of RAC kinase as described in the literature, from any species. Human RAC-PK is preferred. Human RAC-PKa is represented in SEO ID No. 3. Domains of RAC kinase are the individual functional portions thereof, such as the PH domain, the catalytic domain and the C-terminal domain. Fragments of RAC, which include domains of RAC, are functionally active portions of the RAC kinase protein which may be used in the present invention in place of RAC. Fragments of RAC-PK are preferably the domains thereof, advantageously the PH domain, the catalytic domain and the C-terminal domain. In each case, the terminology used embraces mutants and derivatives of RAC-PK and its fragments which can be created or derived from the naturally-occurring protein according to available technology. For instance, nucleic acids encoding RAC-PK may be mutated without affecting the nature of the peptide encoded thereby, according to the degeneracy of the amino acid code. Moreover, conservative amino acid substitutions may be made in RAC-PK or a fragment thereof, substantially without altering its function. Further additions, deletions and/or substitutions which improve or otherwise after the function of RAC-PK or a fragment thereof are envisaged and included within the scope of the invention.

The antibiotic wortmannin, which is known to inhibit phosphatidylinositol 3-OH kinase (PI-3K) activation, targets signal transduction and indirectly inactivates *inter alia* RAC, possibly via PI-3K. Wortmannin has been indicated in the treatment of neoplastic conditions.

However, the broad involvement of the various isoforms of RAC-PK in mitogenic signal transduction as well as insulin-dependent signalling has hitherto not been known. We have now shown that RAC-PK is involved in the regulation of both GSK3 and IMPDH, a factor involved in growth control. It can be concluded, therefore, that RAC-PK plays a central role in growth control.

Therapeutic agents according to the invention may be formulated conventionally, according to the type of agent. Where the agent is a salt, such as vanadate, it is conveniently formulated in aqueous solution at neutral pH and administered orally at room temperature. In the case of a peptide medicament, such as RAC-PK itself, more elaborate delivery techniques, such as liposomal delivery, may be required in order to introduce the peptide into target calls. Delivery systems for peptide therapeutics are documented in the art.

The identification of RAC-PK as a major mediator in growth control permits the design of screening systems to identify putative therapeutic agents for use in treating anomalies of growth control.

The screening may be carried out using complete RAC kinase or a fragment thereof. In particular, it has been shown that the PH domain of RAC kinase is important in mediating many of its effects, as set out, for example, in UK patent application 9525703.6 (Ciba-Geigy AG), filed 15th December 1995. Moreover, as disclosed hereinbelow, RAC-PK interacts with IMPDH via the PH domain. The interaction is not observable in the yeast two-hybrid system if complete RAC-PK is used, although *in vitro* binding of RAG-PK to IMPDH occurs.

Interactions also occur between other fragments of RAC-PK and its physiological targets and regulators. For example, GSK3 binds to RAC-PK via the catalytic domain, as evidenced by the phosphorylation of GSK3 by RAC. CTBP, on the other hand, does not bind the catalytic or PH domains but binds specifically to the carboxy terminal domain of RAC.

Preferably, therefore, the invention includes incubating the compound to be screened with a fragment of RAC, which is advantageously the PH domain, the catalytic domain or the carboxy terminal domain.

RAC-PK fragments for use in the method of the present invention may be in the form of isolated fragments, or in the form of the fragment complexed with further polypeptides. For example, in the case of the two-hybrid system, the fragment is complexed to a DNA binding or transcriptional activation domain derived from another protein, such as the yeast activator GAL4.

Moreover, the RAC-PK used in the method of the invention may be in the form of a mutant thereof, for example a constitutively activated kinase. An important activating residue is T308, present in the so-called T-loop between subdomains 7 and 8 of the kinase. A general guide to kinase structure is given in Woodgett (1994), *Protein Kinases,* IRL Press, UK. Substitution of T308 with aspartic acid results in a clear increase in basal activity of the kinase, which however retains a potential for further activation. The invention therefore provides a RAC kinase protein in which Thr308 has been mutated to Asp.

Preferably, Ser473 is additionally mutated to Asp. Phosphorylation of this residue is required for full activation of RAC-PK *in vivo,* and the T308/S473 double mutant (both residues converted to Asp) shows a constitutive activity 18-fold higher than native RAC-PK. The double mutant is not susceptible to further activation.

The mutations may be carried out by means of any suitable technique. Preferred, however, is *in vitro* site-directed mutagenesis of a nucleotide sequence encoding RAC and subsequent expression of RAC in a recombinant DNA expression system. This method is an in vitro mutagenesis procedure by which a defined site within a region of cloned DNA can be altered (cf. the review articles of M.J. Zoller and M. Smith, Methods Enzymol. (1983), 100, 468; D. Botstein and D. Shortle, Science (1985), **229**, 1193). Methods for site-directed mutagenesis are well known to those of skill in the art, as exemplified by Sambrook (1989): Molecular Cloning - A Laboratory Manual, Cold Spring Harbor, NY, USA, and the number of commercially available *in vitro* mutagenesis kits.

The compound to be screened may be present in essentially pure, uncomplexed form, or may be complexed with chemical groups or further polypeptides. In the case of the two hybrid system, it is complexed to a DNA binding or transcriptional activation domain, in order to complement the PH domain.

Isolated PH domain for use in the present invention may be prepared as set forth in our UK patent application 9525705.1 (Ciba-Geigy AG), filed 15th December 1995. Where a small quantity of PH domain suffices, however, PH domain may be obtained by expressing a nucleic acid sequence encoding it in bacterial cell culture in the form of a fusion protein which is subsequently cleaved according to techniques known in the art. For example, amino acids 1-131 of RAC, which encode the PH domain, may be expressed as a fusion protein, advantageously with glutathione-S-transferase (GST), subsequently cleaving the fusion protein with thrombin and isolating the domain by protein purification techniques such as FPLC. This method gives a relatively small yield of pure soluble PH domain.

Carboxy and kinase domains are likewise advantageously synthesised as fusion proteins, for instance as GST fusions.

RAC-PK or a fragment thereof for use in the present invention may be prepared as set forth in UK patent application 9525702.8. Alternatively, RAC-PK may be expressed in recombinant cell culture. Baculovirus vectors, specifically intended for insect cell culture, are especially preferred and are widely obtainable commercially (e.g. from invitrogen and Clontech). Other virus vectors capable of infecting insect cells are known, such as Sindbis virus (Hahn *et al.,* (1992) PNAS (USA) **89**, 2679-2683). The baculovirus vector of choice (reviewed by Miller (1988) Ann. Rev. Microbiol. **42,** 177-199) is *Autographa californica* multiple nuclear polyhedrosis virus, AcMNPV.

Typically, the heterologous gene replaces at least in part the polyhedrin gene of AcMNPV, since polyhedrin is not required for virus production. In order to insert the heterologous gene, a transfer vector is advantageously used. Transfer vectors are prepared in *E. coli* hosts and the DNA insert is then transferred to AcMNPV by a process of homologous recombination. Baculovirus techniques useful in the present invention are standard and well known in the art, and are reviewed in O'Reilly *et al.,* (1994) Baculovirus expression vectors; A laboratory manual, Oxford University Press Inc., NY, USA, as well as in literature published by suppliers of commercial baculovirus kits (e.g. Pharmingen).

Incubation conditions will vary according to the precise method used to detect the interaction between the PH domain and the screened compound. In the case of transcription activation detection systems such as the yeast two-hybrid system, incubation conditions are suitable for gene transcription, such as those prevailing inside a living cell. Other detection systems, however, will require different incubation conditions. For example, if the detection of interaction is based on relative affinity in a chromatographic assay, for example as is known in affinity chromatography, conditions will be adjusted to promote binding and then gradually altered, such that the point at which the screened compound no longer binds to the RAC-PK PH domain may be determined.

The detection method may employ the natural fluorescence of Trp²² in the RAC-PK PH domain, which is inhibited by certain interactions with the domain, as set forth in UK patent application 9525703.6. Briefly, fluorescence of the amino-terminal Trp residue in the PH domains of certain PH domain containing proteins may be detected by exciting the molecule to fluoresce at the appropriate frequency and monitoring the emission. The N-terminal Trp²² of RAC, for example, fluoresces at 345nm when excited at 290nm. Techniques for monitoring protein fluorescence are widely known in the art. We have shown that the PH domain of RAC-PK binds phospholipid with high affinity, which suggests that RAC-PK may be membrane-bound *in vivo* via the PH domain. Binding of phospholipid to the RAC-PK PH domain quenches the natural fluorescence of the N-terminal tryptophan at position 22 (Trp²²). Interaction of the PH domain with the cell membrane is believed to be important for the stable interaction of RAC-PK with membrane bound partners in signalling pathways, such that disruption of this interaction will lead to modulation of the signalling effect through the dissociation of the signalling molecule from the cell membrane. The modulation could be either down-regulating, for example if the otherwise stable interaction of the molecule with membrane-bound partners is a stimulatory interaction, or up-regulating, in the event that the interaction is an inhibitory interaction. Accordingly, a compound which is a candidate modulator of signal response may be screened for by means of a method comprising the steps of:
(a) incubating the compound with the PH domain of a signaling molecule which is capable of fluorescing;
(b) determining the phospholipid-induced modulation in the fluorescence of the PH domain, an alteration of the fluorescence in the presence of the compound being indicative of a functional interaction between the compound and the PH domain.

In this case, the incubation conditions will be adjusted to facilitate the detection of fluorescence at 345nm when the PH domain is excited at a frequency of 290nm.

incubation according to the invention may be achieved by a number of means, but the basic requirement is for RAC-PK or a fragment thereof and the screened compound to be able to come into contact with each other. This may be achieved by admixing RAC-PK or a fragment thereof and the compound, or by producing them *in situ,* such as by expression of nucleic acids encoding them. Where the RAC-PK or RAC-PK fragment and/or the compound are in the form of fusions with other polypeptides, they may be expressed as such *in situ*.

Preferably, the method of the invention is based on a two-hybrid system. Such systems detect specific protein:protein interactions by exploiting transcriptional activators having separable DNA-binding and transcription activating domains, such as the yeast GAL4 activator. A reporter gene is operatively linked to an element responsive to the transcriptional activator being used, and exposed to RAC-PK or a fragment thereof and the compound to be screened, one of which is complexed to the transcription activating domain of the transcriptional activator and the other of which is joined to the DNA binding domain thereof. If there is a specific interaction between RAC-PK or a fragment thereof and the compound, the DNA binding and transcription activating domains of the transcriptional activator will be brought into juxtaposition and transcription from the reporter gene will be activated.

Alternatively, the detection may be based on observed binding between RAC-PK or a fragment thereof, such as its PH domain or its catalytic domain, and the screened compound, or a fragment thereof. For example, the interaction between RAC-PK and the insulin mediator GSK3 is detected hereinbelow by monitoring the interaction of a peptide surrounding the major phosphorylation site of GSK3 known to be responsible for its inactivation with RAC kinase. Preferably, the method of the invention provides that the substrate in step (b) comprises the peptide GRPRTSSFAEG and that the serine residue closer to the C-terminus of the peptide is phosphorylated. In a similar manner, the involvement of RAC-PK on the activation or inactivation of a particular compound may be screened for by monitoring the interaction of a portion thereof known to be involved in modulation events with RAC.

RAC-PK or a fragment thereof may be used to screen for compounds which bind thereto by incubating it with the compound to be screened and subsequent "putting down" RAC-PK complexes with a RAC-specific antibody. Antibodies suitable for immunoprecipitation or immune-affinity chromatography may be prepared according to conventional techniques, known to those of ordinary skill in the art, and may be monoclonal or polyclonal in nature. For example, see Lane, *et al.,* (1992) EMBO J., 11, 1743-1749. After the RAC-compound complex has been isolated by affinity, the compound may be dissociated from the RAC-PK antibody and characterised by conventional techniques.

The interaction of RAC-PK or a fragment thereof with the screened compound may also be observed indirectly. For example, an inhibitor or activator of RAC-PK function may be detected by observing the effects of RAC-PK on a substrate in the presence or absence of the compound.

The activity of RAC-PK or the catalytic domain thereof may be assessed by means of a kinase activity assay, employing a substrate for the kinase. For example, myelin basic protein may be used, in accordance with established assay procedures. Physiological substrates, such as GSK3, may also be used. Alternatively, RAC-PK activity may be assessed by determining the degree of activating phosphorylation of RAC-PK itself Advantageously, phosphorylation on residues normally implicated in kinase activation is assessed. RAC-PK, as disclosed in EP 877 818 A1, is preferentially activated by phosphorylation at serine and threonine residues.

Accordingly, a preferred embodiment of the invention provides a method for the screening of an activator of RAC-PK function, wherein the compound in step (c) of the method is selected if it increases the amount of phosphorylated residues of said substrate. Alternatively, a method for the screening of an inhibitor of RAC-PK function is provided, wherein the compound in step (c) of the method is selected if it decreases the amount of phosphorylated residues of the substrate.

The assay of the invention may be used to measure the direct effect of the candidate compound on RAC, or it may be used to determine the effect of the compound on a kinase acting upstream thereof in a signalling pathway. In the latter situation, RAC-PK acts as a substrate for the upstream kinase and the activity of the upstream kinase is assessed by determining the phosphporylation state or the activity of RAC-PK

In order to obtain a meaningful result, the activity of RAC-PK exposed to the candidate immunosuppressive or antiproliferative agent should be compared to the activity of RAC-PK not exposed to the agent, a modulation of RAC kinase activity being indicative of potential as a modulator of cell proliferation and/or insulin signal transduction.

Promising compounds may then be further assessed by determining the properties thereof directly, for instance by means of a cell proliferation assay. Such an assay preferably involves physical determination of proliferation in calls which have been subjected to kinase activation by a phosphatase inhibitor, exposed to the candidate RAC-PK modulator and optionally subsequently stimulated with a mitogen, such as a growth factor, IL-2 or PMA Mire simply, the assay may involve exposure of unstimulated cells to the candidate modulator, followed by stimulation with a phosphatase inhibitor.

The invention further comprises the use of RAC-PK or a fragment thereof in a screening system. The screening system is preferably used to screen for compounds which are modulators of insulin activity, particularly where that activity is related to glycogen metabolism or cell proliferation.

In a still further embodiment, the invention provides a compound which interacts directly or indirectly with RAC-PK or a fragment thereof. In the case of indirectly acting compounds, agents such as insulin and wortmannin are excluded. Such a compound may be inorganic or organic, for example an antibiotic, and is preferably a proteinaceous compound involved in intracellular signalling. For example, the compound may be CTBP (SEQ ID Nos. 1 and 2).

Compounds according to the invention may be identified by screening using the techniques described hereinbefore, and prepared by extraction from natural sources according to established procedures, or by synthesis, especially in the case of low molecular weight chemical compounds. Proteinaceous compounds may be prepared by expression in recombinant expression systems, for example a baculovirus system as described hereinbefore or in a bacterial system, for example as described in UK patent application 9525705.1. Proteinaceous compounds are mainly useful for research into the function of signalling pathways, although they may have a therapeutic application.

Low molecular weight compounds, on the other hand, are preferably produced by chemical synthesis according to established procedures. They are primarily indicated as therapeutic agents. Low molecular weight compounds and organic compounds in general may be useful as insulin mimics or antiproliferative agents.

The invention is further described, for the purposes of illustration only, in the following examples.

### Example 1

### Specific Interaction of RAC-PK with IM PDH

### (a) Bacterial and Yeast Strains

All yeast strains and plasmids for two-hybrid experiments are obtained from Clontech (Palo Alto, CA) as components of the MATCHMAKER Two Hybrid System or from Dr. D. Nathans (Howard Hughes Medical Institute, Baltimore, ML). Yeast strains SFY526 *(MATa, ura3-52, his3-200, ade2- 101, lys2-801, trp1-901, leu2-3, 112, can*^{*r*}*, gal4-542, gal80-538, URA3::GAL1-lacZ),* HF7c *(MATa, ura3-52, his3-200, lys2-801, ade2-101, trp1-901, leu2-3, 112, gal4-542, gal80-538, LYS2::GAL1-HIS3, URA3::(GAL4 17-mer)*_{*3*}*-CYC1-lacZ)* and PCY2 [Chevray and Nathans, 1992] *(MATα, his3-200, ade2-101, lys2-801, trp1-63, leu2-3, gal4-542, gal80-538, URA3::GAL1-lacZ)* are used to assay for protein-protein interactions. Yeast strain HF7c is used for library screening. SFY526 and PCY2 have the upstream activating sequence and TATA sequence of the GAL1 promoter fused to the *lacZ* gene. In HF7c, HIS3 is fused to a GAL1 promoter sequence and LacZ is fused to three copies of a 17-mer GAL4 consensus sequence plus the TATA sequence of the CYC1 promoter. Both HIS3 and LacZ are responsive to GAL4 activation. Yeast techniques including transformation are performed according to the instructions in the MATCHMAKER Two Hybrid System and as described [Ausubel, *et al.* (1994) *Current Protocols in Molecular Biology.* John Wiley and Sons, New York, NY]. The bacterial strains XL1-blue (Statagene) and JM109 are employed in the cloning of plasmids and the production of GST fusion proteins. The bacterial strains JM109(DE3), BL21 (DE3)pLysS and BL21 (DE3)pLysE (Invitrogen) are used for the production of (HIS)₆-tagged proteins. General molecular biological techniques are performed as previously described [Sambrook, *et al.* (1989) *Molecular Coning: A Laboratory Manual.,* 2nd Ed., Cold Spring Harbor Laboratory, Cold Spring Harbor, NY; Davis, *et al.* (1986) *Basic Methods in Molecular Biology.* Elsevier Science Publishing Co., New York, NY].

### (b) Plasmid Construction

Yeast vector plasmids containing the GAL4 DNA binding domain (amino acids 1-147, pGBT9) and the GAL4 activation domain (amino acids 768-881, pGAD424) as well as the control plasmids pCL1 (full-length GAL4 gene), pVA3 (p53 gene), pTD1 (SV40 large T antigen), and pLAM5' (human lamin C gene) are from Clontech. The yeast vector pPC62, containing the GAL4 DNA binding domain, is from Dr. D. Nathans. The glutathione-*S*-transferase (GST) fusion vector pGEX-2T is from Pharmacia. The baculovirus transfer vector (pVL1392) and the (HIS)₆-tag vector (pRSET-A) are from invitrogen. pGBT-PH127, pGBT-PH150, pGBT-PHI-III, and pGBT-PHIII-VI contain in-frame fusions of amino acids 1-127, 1-150, 1-47, 47-127 of the human RACα PH domain, respectively, with the GAL4 DNA binding domain. They are constructed by subcloning PCR fragments generated with specific oligonucleotides into the *Eco*RI*-Bam*Hl sites of pGBT9. pGEX-PH131, pGEX-PH-KIN, pGEX-PH-KIN-CT, pGEX-KIN-CT, pGEX-KIN, and pGEX-CT contained in-frame fusions of amino acids 1-131, 1-411, 1-480, 147-480, 147-411, 411-480 of human RACα, respectively, with GST. They are constructed by subcloning PCR fragments generated with specific oligonucleotides into the *Bam*HI-*Eco*RI sites of pGEX-2T. pGBT-PH-KIN, pGBT-PH-KIN-CT, pGBT-KIN-CT, pGBT-KIN, and pGBT-CT contain in-frame fusions of amino acids 1-411, 1-480, 147-480, 147-411, 411-480 of human RACα, respectively, with the GAL4 DNA binding domain. They are constructed by subcloning the appropriate *Bam*HI-*Eco*RI fragments from the corresponding pGEX constructs into the *Pst*l*-Xba*l sites of pPC62 using *Pst*l*-Bam*Hl and *Eco*Rl*-Xba*l adapters. The *Xho*l*-Xba*l fragments from the resultant pPC62 plasmids are then isolated and subcloned into the *Xho*l*-Eco*RI sites of pGBT9 using a *Xba*l*-Eco*RI adapter. pGAD-IMPDH1-481, pGAD-IMPDH1-427, pGAD-IMPDH1-325, pGAD-IMPDH28-514, pGAD-IMPDH70-514, pGAD-IMPDH1-40, and pGAD-IMPDH428-514 contain in-frame fusions of amino acids 1-481, 1-427, 1-325, 28-514, 70-514, 1-40, 428-514 of human IMPDH type II, respectively, with the GAL4 activation domain. They are constructed by subcloning PCR fragments generated with specific oligonucleotides into the *Bam*HI*-Sat*l sites of pGAD424, pGEX-IMPDH contains an in-frame fusion of the complete human IMPDH type II with GST. It is constructed by subcloning the *Sma*l-*Xho*l IMPDH fragment from pGADGH-IMPDH into the *Sma*l site of pGEX-2T using a *Xho*l-*Sma*l adapter. pVL1392-hRACα contained the *Eco*RI fragment from WI38xRAC71 [Jones, *et al.* (1991) *Proc. Natl. Acad. Sci. U. S. A*. **88**, 4171-4175] encompassing the full coding region of human RACα. pRSET-PHQKKK contains an in-frame fusion of amino acids 1-116 of human RACα with an amino-terminal (HIS)₆-tag, and the addition of three lysines at the carboxy terminus. It is constructed by subcloning an *Nde*l*-Pf*Ml fragment from pRK-RAC [Jones, *et al.* (1991) *Proc. Natl. Acad. Sci. U. S. A*. **88**, 4171-4175] into the *Bam*Hl-*Eco*RI sites of pRSET-A using *Bam*HI*-Nde*l and *Pfl*MI-*Eco*RI adapters. All plasmid constructions are confirmed by restriction fragment analysis and sequencing.

### (c) Library Screening

To determine if RAC's PH domain could interact with other proteins we fuse it to the GAL4 DNA binding domain and screen a HeLa cDNA library fused to the GAL4 transcriptional activation domain in the yeast reporter strain HF7c. The human HeLa S3 MATCHMAKER cDNA library is purchased from Clontech. pGBT-PH127 is transformed into HF7c with and without the control plasmids (pGAD424, pCL1, pTD1). Colonies from this transformation are tested for HIS3 and LacZ expression to confirm that the PH domain alone does not activate transcription. The HF7c transformant containing just pGBT-PH127 is then transformed with enough of the HeLa S3 cDNA library inserted into the two-hybrid activation vector pGADGH to produce 1.0 X 10⁶ yeast Leu⁺/Trp⁺ transformants. Doubly transformed cells are plated onto Leu⁻, Trp-, His- plates and incubated at 30°C for 3-8 days. Positive colonies are picked, restreaked onto triple minus plates and assayed for LacZ activity by the filter assay. Library clones that are His⁺ and LacZ⁺ are then cured of the pGBT-PH127 plasmid and tested again for His auxotrophy and LacZ activity. Cured clones that are negative in both assays are then mated to transformants of PCY2 containing either pGBT9, pGBT-PH127, pLAM5' or pTD1. The activation clones corresponding to the diploids which become positive for both His auxotrophy and LacZ activity only in the presence of pGBT-PH127 are chosen for sequencing.

In our screen of 1.0 X 10⁶ primary transformants we identify 37 clones which show specific interaction with RAC's PH domain, by activation of the reporters for His auxotrophy and LacZ activity. These clones could be subdivided into 6 different cDNA classes, based on the size of the cDNA insert. Upon sequencing all clones were found to encode human IMPDH type II inclusive of the initiator methionine through to the termination codon of the previously cloned cDNA [Collart, F. R., and Huberman, E. (1988) *J. Biol. Chem.* **263**, 15769-15772].

The interaction requires a complete PH domain as constructs containing either subdomains I-III (amino acids 1-47) or subdomains IV-VI (amino acids 47-127) alone do not show any interaction with IMPDH. The lack of interaction with subdomains IV-VI is significant as this region has previously been shown to interact weakly with the βγ-subunits of heterotrimeric G-proteins. This interaction of IMPDH and RAC's PH domain is however inhibited in the two-hybrid system with constructs containing RAC's kinase domain juxtaposed to RAC's PH domain as occurs in its natural context. This could be due to an intramolecular interaction of RAC's PH domain with itself or another region of RAC. However, inclusion of amino acids between the PH and kinase domains (including the first four amino acids of the kinase domain) didn't inhibit the interaction. We also fuse RAC's PH domain to the GAL4 activation domain to test if it could interact with any of the human RACα GAL4 DNA binding constructs. We can detect no such interaction, indicating that the PH domain does not self associate or form a complex with other regions of the RAC-PK molecule in this system. The inhibition of interaction observed above would thus appear to be due to steric hindrance.

We construct nested amino and carboxyl-terminal deletions of IMPDH to determine the region of the molecule responsible for interaction with RAC's PH domain. This indicates that an almost intact IMPDH molecule is required for the interaction. The amino-terminal boundary of the PH interaction domain is found to lie between amino acids 28 and 70, while the carboxyl-terminal boundary lies between amino acids 427 and 481.

### (d) In Vitro Binding Studies

To test if IMPDH could interact directly with RAC's PH domain we employ an *in vitro* binding assay system using GST fusions. GST fusions produced from the plasmids pGEX-2T, pGEX-PH131 and pGEX-IMPDH are expressed in *E. coli* XL-1 blue cells by induction with 0.1 mM IPTG for 2 hours at 24°C. The fusion proteins are purified as described [Smith, D. B., and Johnson, K. S. (1988) *Gene* **67,** 31-40] except that the cells are lysed in a French Press. The human RACα PH domain (HIS)₆ tagged fusion produced by BI21 (DE3)pLysS cells transformed with pRSET-PHQKKK is expressed and purified as described [UK patent application 9525705.1]. Briefly, cells are induced with 0.2 mM IPTG for 2 hours at 24°C before harvesting. Cell pellets are lysed in a French Press and the soluble PH domain is purified sequentially on Ni(II) affinity, cation exchange and gel filtration columns. Binding studies are performed using GST fusions (2.5 µg) coupled to glutathione-agarose beads in binding buffer (20 mM phosphate buffer pH 7.2, 150 mM NaCl, 1% Triton X-100, 5 mM DTT) containing 2.5 µg of (HIS)₆-tagged PH domain or baculovirus produced human RACα in a total volume of 100µl. The samples are incubated at 4°C for 1-2 hours with agitation every 5 min. The beads are then washed three times with buffer (20 mM phosphate buffer pH 7.2, 150 mM NaCl) before being analysed by SDS-PAGE and stained with coomassie blue R-250 (for (HIS)₆-tagged PH domain binding) or SDS-PAGE followed by Western blot analysis using a human RACα specific antiserum (for human RACα binding) [Jones *et al., Op. Cit*]. The secondary antibody is a horseradish peroxidase coupled anti-rabbit antibody (Amersham) which is detected using the ECL method (Amersham) by autoradiography. In this assay we see that the (HIS)₆-tagged PH domain can bind to the GST-IMPDH fusion but not to GST alone.

We also employ this assay system to test if full-length baculovirus purified human RACα could directly interact with IMPDH. Full-length human RACα is expressed and purified from the baculovirus system as described [see UK patent application 9525702.8]. Briefly, a baculovirus is constructed by co-transfection of Sf9 cells with pVL1392-hRACα and wild-type baculovirus AcMNPV DNA and purified by limiting dilution and detected by dot-blot hybridisation. The purified virus is used to produced human RACα in Sf9 cells. The human RACα is purified by sequential anion exchange, phospho-cellulose and gel filtration chromatography. Here we see specific interaction of the full-length RAC-PK molecule with GST-IMPDH and not GST alone or the GST-PH fusion.

### (e) In vivo Pull-down Assay

Using GST-IMPDH in a pull-down assay with MCF-7 cell extracts we see a specific association of human RACα with the GST-IMPDH and not with GST. MCF-7 cells are lysed in buffer (50 mM Tris-HCI pH 8.0, 120 mM NaCl, 1% NP-40, 1mM EDTA, 1 mM EGTA, 30 mM pNPP, 25 mM β-glycerol phosphate, 15 mM PPi, 25 mM NaF, 1 mM vanadate, 20 µM PAO, 1 mM benzamidine, 0.1 mM PMSF) using twelve strokes of a dounce homogeniser. Soluble protein from the supernatant of lysates centrifuged at 14,000 x g for 15 min at 4°C are added to GST, GST-PH or GST-IMPDH protein (5 µg) attached to glutathione beads and incubated at 4°C for 2 hours with continuous agitation. The beads are then washed four times with lysis buffer before being analysed by Western blotting as described above with the human RACα specific antiserum [Jones *et al., Op. Cit.;* (1991) *Cell Reg.* **2**, 1001-1009] or an IMPDH specific antiserum [Collart, F. R., and Huberman, E. (1988) *J. Biol. Chem.* **263**, 15769-15772]. We could also perform the converse experiment, pulling down IMPDH from cell lysates using the GST-PH domain fusion protein. Thus we have shown the existence of an association between human RACα and human IMPDH type It in three heterologous systems.

### (f) Enzyme Assays

We then assay the effect of this interaction on the activity of IMPDH. The addition of soluble PH domain as a GST fusion produced an activation of IMPDH compared to the addition of GST alone. Assays for IMPDH activity are performed essentially as described [Antonio, L. C., and Wu, J. C. (1994) *Biochem.* **33**, 1753-1759] monitoring the production of XMP by absorbance at 286 nm. The IMPDH is produced as a GST fusion purified on glutathione beads and then eluted as soluble protein with reduced glutathione. IMPDH activity is tested in the presence of either soluble GST (produced from pGEX-2T) or PH domain (produced from pGEX-PH131) at a molar ratio of IMPDH to GST/PH domain of 1:5, RAC kinase assays with the baculovirus produced human RACα using various substrates (myelin basic protein, GST or GST-IMPDH) are performed essentially as described [Jones *et al., Op. Cit.].* When IMPDH is tested in RAC kinase assays using the baculovirus produced human RACα to see if it is a substrate we could detect no significant phosphorylation of the IMPDH.

### Example 2

### Inhibition of GSK3

Two major kinases known to be involved in regulating the insulin-dependent signalling pathways are MAPKAP kinase-1 and p70^{S6K}. These kinases are respectively inhibited by PD98059 and rapamycin. Both of these agents fail to inhibit phosphorylation of GSK3, suggesting that the kinase responsible for GSK3 inactivation is not MAPKAP kinase-1 or P70^{S6K}.

L6 myotubes are incubated with both compounds and the stimulated with insulin, as follows. Monolayers of L6 cells are grown in 6cm petri dishes to the stage of myotubes, deprived of serum overnight and then incubated for 1 hour in 20mMHepes/NaOH (pH 7.4) / 0.14M NaCI /5mM KCI / 2.5mM MgSO₄ / mM CaCl₂ / 25mM glucose (HBS buffer), in the presence or absence of 50µM PD98059 or 100µM LY294002. 2mM 8-Br-cAMP or 0.1 µM rapamycin, when added, are included for the last 15min. The cells are stimulated for 5min. with 0.1µM insulin, or for time courses of up to 10 min. The medium is removed by aspiration, the cells placed on ice and lysed in 0.2ml of ice-cold buffer A [50mM Tris-HCI (pH7.5, 20°C) / 1mM EDTA / 1mM EGTA / 1% (w/v) Triton X-100 / 1mM sodium orthovanadate / 10mM sodium glycerophosphate / 50mM sodium fluoride / 5mM sodium pyrophosphate / 2µM microcystin / 0.1% (v/v) 2-mercaptoethanol / leupeptin 4µg/ml, 1 mM benzamidine, 1mM phenylmethane sulphonyl fluoride, 30 µg/ml aprotinin, 30 µg/ml antipain, 10 µg/ml pepstatin.

Precipitation of p42^{MAPK}, MAPKAP kinase-1 or GSK3 from the cell lysates by immunoprecipitation and analysis of their activity with specific protein or peptide substrates (Cross *et al.,* (1994) Biochem. J. 303, 21-26) shows that insulin inactivation of GSK3 is not affected by agents (2mM 8-Br-cAMP or 0.1µM rapamycin) which inhibit classical MAP kinase or p70^{S6K} signalling pathways.

In order to identify the kinase which inhibits GSK3 in the presence of rapamycin and PD98059, cells are lysed as above and the cell lysates (0.3mg) chromatographed on a 5 x 0.16cm column of Mono Q (Sutherland *et al.,* (1994) FEBS Lett. 338, 37-42) except that the buffer additionally contains 1 mM EGTA, 0.1 mM sodium orthovanadate and 0.5% (w/v) Triton X-100.

The fractions (0.05ml) assayed with the synthetic peptide GRPRTSSFAEG, which corresponds to the sequence of GSK3 surrounding the serine (bold type) whose phosphorylation triggers the inactivation of GSK 3α (Ser²¹) and GSK3β (Ser⁹). Three peaks of activity which result in phosphorylation of this peptide are eluted. These peaks are absent if insulin stimulation is not given, or if cells are incubated with 0.1 µM wortmannin prior to insulin stimulation. The inactivating effect of insulin on GSK3 is known to be prevented by administering this concentration of wortmannin, or 100µM LY294002, a structurally unrelated inhibitor of PI-3K.

All three peaks of phosphorylating activity can be immunoprecipitated with an anti-RAC antibody using a polyclonal rabbit antiserum directed against the peptide FPQFSYSASSTA raised by injecting rabbits subcutaneously with the peptide and purified by precipitation using 50% (NH₄)₂SO₄ followed by affinity chromatography on RAC-peptide coupled Affigel® 10 column (Bio-Rad).

In contrast, immunoprecipitating with an anti-MAPKAP kinase-1 antibody fails to deplete any peptide phosphorylating activity from the Mono Q column.

In order to determine that complete GSK3 can be inactivated by RAC, GSK3α and GSK3β are partially purified from rabbit skeletal muscle (Sutherland *et al., Op. Cit.)* and assayed with a specific peptide substrate (Cross *et al., Op. Cit.).* Each GSK3 isoform is diluted to 15U/ml and GSK3 activity measured after incubation for 20min with MgATP in the presence or absence of RAC. The incubation is the made 20mM in EDTA to stop the kinase reaction, incubated for 20min with 5 mU/ml PP2A₁ to reactivate GSK3, made 2µM in okadaic acid to inactivate PP2A₁ and then assayed for GSK3 activity.

In the absence of RAC, GSK3 is stably active throughout the experiment. Otherwise, however, RAC-PK successfully inhibited GSK3 activity and this inactivation was sensitive to PP2A₁, which restored GSK3 activity. The absence of insulin stimulation, the presence of wortmannin or the disruption of RAC-PK immunoprecipitation by incubation of the anti-RAC antibody with the peptide immunogen all result in a lack of GSK3 inactivation in this experiment.

### Example 3

To determine if RAC's kinase domain with its carboxyl-terminal extension could interact with other proteins we fused it to the GAL4 DNA binding domain and screened a HeLa cDNA library fused to the GAL4 transcriptional activation domain in the yeast reporter strain HF7c following the procedure of Example 1. Briefly, amino acids 147-480 of RACα are fused in frame to GST in the expression vector pGEX-2T (see Example 1). Appropriate BamHl-*E*coRI fragment is then subcloned into the *Pst*l-Xbal sites of yeast vector pPC62 (Dr. D. Nathans) using *Pst*l*-Bam*HI and *Eco*RI*-Xba*l linkers in order to create a GAL4 DNA binding domain-RAC kinase and C-terminal domain fusion. *Xho*l*-Xba*l fragments are then subcloned into pGBT9 (Clontech). The HeLa S3 MATCHMAKER cDNA library is used as before.

In our screen of 1.5 X 10⁶ primary transformants we identify seven clones which show specific interaction with RAC's kinase domain plus the carboxyl-terminal extension, by activation of the reporters for His auxotrophy and LacZ activity. A detailed analysis of the specific region of RAC-PK that the clone interacts with shows that the carboxyl-terminal 69 amino acids are all that is required to confer the interaction. We thus denote this molecule CTBP, for Carboxyl-Terminal Binding Protein. None of the clones shows an interaction with the kinase domain alone. This interaction is seen in all constructs containing the carboxyl-terminal extension including a full-length RAC-PK construct, indicating that the interaction is not inhibited by another region of the RAC-PK molecule. Interestingly, the C-terminal domain of RAC-PK is phosphorylated in response to insulin activation, suggesting a role for CTBP as a modulator of insulin action.

All seven specific interacting clones contain identical cDNA inserts of 1.3 kb in length with an ALU repeat of ~300 nt at the 3' end (SEQ ID No. 1). Searches of the Gene-EMBL nucleotide database using the cDNA sequence without the ALU repeat with the FASTA programme (GCG Package) identify no significant homologies. The deduced amino acid sequence of the CTBP cDNA produces a short 47 residue polypeptide rich in alanines (21%) and arginines (21%). Searches of the PIR, Swiss-Prot, and GP protein databases using FASTA (GCG Package) and the Gene-EMBL nucleotide database using TFASTA (GCG Package) reveal no significant homologies to the CTBP protein sequence.

The sequence of CTBP identified is believed to represent the 3' end of the complete CTBP molecule.

To test if the novel protein CTBP could interact directly with RAC-PK we employ an *in vitro* binding assay system using GST fusions, as described in Example 1. In this assay we see specific interaction of the full-length RAC, produced in the baculovirus system with GST-CTBP and not GST alone.

To test if this interaction occurs *in vivo* we employ a pull-down assay using the GST-CTBP fusion protein and MCF-7 cell extracts, as described in Example 1. Here we see the specific association of the MCF-7 RACα with the GST-CTBP but not GST alone.

## Claims

1. A method of screening for compounds interacting with RAC-PK comprising the steps of:
(a) incubating RAC-PK or a functional fragment thereof and the compound to be screened,
(b) measuring a kinase activity of RAC-PK or a functional fragment thereof using GSK3 as substrate, and
(c) selecting the compound, if said substrate has a different amount of phosphorylated residues as compared to control reaction without said substrate.

2. The method of claim 1 wherein human RAC-PK or a functional fragment thereof is used.

3. The method of claim 1 or 2 wherein the substrate in step (b) comprises the peptide GRPRTSSFAEG and wherein the serine residue closer to the C-terminus of said peptide is phosphorylated.

4. The method of claim 1, 2 or 3 wherein the functional fragment in step (a) is the catalytic domain of RAC-PK.

5. A method for the screening of an activator of RAC-PK function according to any of claims 1 to 4 wherein the compound in step (c) is selected if it increases the amount of phosphorylated residues of said substrate.

6. A method for the screening of an inhibitor of RAC-PK function according to any of claims 1 to 4 wherein the compound in step (c) is selected if it decreases the amount of phosphorylated residues of said substrate.

7. A method according to claim 5 for the screening of compounds useful in the treatment of diseases where blood sugar levels are excessive.

8. A method according to claim 6 for the screening of compounds useful in the treatment of diseases involving insufficiency in blood sugar levels.

9. A method according to claim 7 for the screening of compounds useful in the. treatment of diabetes.

10. A method according to any of claims 1 to 9 wherein RAC-PK having one or both mutations T308 ->D and S473 -> D is used.

## Patentansprüche

1. Verfahren zum Screenen von Verbindungen, die mit RAC-PK wechselwirken, das die folgenden Schritte umfasst:
(a) Inkubation von RAC-PK oder eines funktionellen Fragments hiervon und der zu screenenden Verbindung,
(b) Messung der Kinaseaktivität der RAC-PK oder eines funktionellen Fragments hiervon mittels GSK3 als Substrat, und
(c) Auswahl der Verbindung, falls das Substrat eine unterschiedliche Menge an phosphorylierten Resten im Vergleich zur Kontrollreaktion ohne Substrat aufweist.

2. Verfahren nach Anspruch 1, worin humane RAC-PK oder ein funktionelles Fragment hiervon verwendet wird.

3. Verfahren nach Anspruch 1 oder 2, worin das Substrat in Schritt (b) das Peptid GRPRTSSFAEG umfasst und worin der Serinrest, der näher am C-Terminus des Peptids liegt, phosphoryliert ist.

4. Verfahren nach Anspruch 1, 2 oder 3, worin das funktionelle Fragment in Schritt (a) die katalytische Domäne von RAC-PK ist.

5. Verfahren zum Screenen eines Aktivators der RAC-PK Funktion gemäß einem der Ansprüche 1 bis 4, worin die Verbindung in Schritt (c) ausgewählt wird, falls sie die Menge an phosphorylierten Resten dieses Substrats erhöht.

6. Verfahren zum Screenen eines Inhibitors der RAC-PK Funktion gemäß einem der Ansprüche 1 bis 4, worin die Verbindung in Schritt (c) ausgewählt wird, falls sie die Menge an phosphorylierten Resten in diesem Substrat verringert.

7. Verfahren nach Anspruch 5 zum Screenen von Verbindungen, die zur Behandlung von Erkrankungen brauchbar sind, bei denen die Blutzuckerspiegel zu hoch sind.

8. Verfahren nach Anspruch 6 zum Screenen von Verbindungen, die zur Behandlung von Erkrankungen brauchbar sind, die zu geringe Blutzuckerspiegel umfassen.

9. Verfahren nach Anspruch 7 zum Screenen von Verbindungen, die zur Behandlung von Diabetes brauchbar sind.

10. Verfahren nach einem der Ansprüche 1 bis 9, worin RAC-PK mit einer oder beiden Mutationen aus T308 → D und S473 → D verwendet wird.

## Revendications

1. Procédé de criblage de composés interagissant avec la RAC-PK, comprenant les étapes consistant à :
(a) faire incuber une RAC-PK ou un fragment fonctionnel de celle-ci et le composé à cribler,
(b) mesurer l'activité de kinase de la RAC-PK ou d'un fragment fonctionnel de celle-ci en utilisant GSK3 comme substrat, et
(c) sélectionner le composé, si ledit substrat contient une quantité différente de résidus phosphorylés par comparaison avec une réaction témoin sans ledit substrat.

2. Procédé selon la revendication 1, dans lequel on utilise une RAC-PK humaine ou un fragment fonctionnel de celle-ci.

3. Procédé selon la revendication 1 ou 2, dans lequel le substrat de l'étape (b) comprend le peptide GRPRTSSFAEG, et dans lequel le résidu sérine plus proche de l'extrémité C dudit peptide est phosphorylé.

4. Procédé selon la revendication 1, 2 ou 3, dans lequel le fragment fonctionnel de l'étape (a) est le domaine catalytique de la RAC-PK.

5. Procédé de criblage d'un activateur du fonctionnement de la RAC-PK conforme à l'une quelconque des revendications 1 à 4, dans lequel le composé de l'étape (c) est sélectionné s'il accroît la quantité de résidus phosphorylés dans ledit substrat.

6. Procédé de criblage d'un inhibiteur du fonctionnement de la RAC-PK conforme à l'une quelconque des revendications 1 à 4, dans lequel le composé de l'étape (c) est sélectionné s'il diminue la quantité de résidus phosphorylés dans ledit substrat.

7. Procédé selon la revendication 5 pour le criblage de composés utiles dans le traitement de maladies dans lesquelles les taux de sucre sanguins sont excessifs.

8. Procédé selon la revendication 6 pour le criblage de composés utiles dans le traitement de maladies faisant intervenir une insuffisance des taux de sucre sanguins.

9. Procédé selon la revendication 7 pour le criblage de composés utiles dans le traitement du diabète.

10. Procédé selon l'une quelconque des revendications 1 à 9, dans lequel on utilise une RAC-PK ayant l'une des mutations T308→D et S473→D ou les deux.
